# EUROPÄISCHE PATENTSCHRIFT

(11) **EP 2 236 104 B1**
(45) Veröffentlichungstag und Bekanntmachung des Hinweises auf die Patenterteilung: **19.06.2013**
(21) Anmeldenummer: 09156927.7
(22) Anmeldetag: 31.03.2009
(51) Int. Cl.: A61B 19/00

(54) **Medizintechnische Navigations-Bildausgabe mit virtuellen Primärbildern und realen Sekundärbildern**
Medicinal navigation image output with virtual primary images and real secondary images
Sortie d'image de navigation médicale dotée d'images primaires virtuelles et d'images secondaires réelles

(43) Veröffentlichungstag der Anmeldung: 06.10.2010
(73) Patentinhaber: BrainLAB AG, 85622 Feldkirchen (DE)
(72) Erfinder: Schmidt, Robert, 80805, München (DE)
(74) Vertreter: Schwabe - Sandmair - Marx

(56) Entgegenhaltungen:
- US-A- 5 749 362
- US-A- 5 765 561
- US-A1- 2002 075 201
- US-A1- 2002 082 498
- US-A1- 2007 183 637
- US-A1- 2007 238 981
- US-A1- 2008 183 068
- US-B1- 6 231 526

## Beschreibung

Die Erfindung betrifft das technische Gebiet der medizinischen Navigations-Bildausgabe. Die medizintechnische Navigation wird im Rahmen der bildgeführten Chirurgie eingesetzt und unterstützt Chirurgen bei der optimalen Positionierung ihrer Instrumente, wobei z.B. auf vorab akquirierte Patientenbilddaten zurückgegriffen wird. Im Ergebnis erhält der behandelnde Arzt Unterstützung durch eine Bildausgabe, beispielsweise einen Monitor, auf dem er erkennen kann, wo sich sein Instrument oder dessen funktioneller Abschnitt in Relation zu bestimmten Patientenkörperteilen - interessierenden Regionen - befindet. Speziell ist diese Technologie bei der minimal invasiven Behandlungsart von Vorteil, wo Instrumente beispielsweise durch kleine Öffnungen in der Patientenhautoberfläche eingeführt werden und mit Hilfe der Navigation erkennbar wird, wo sich die Instrumente gegenüber bestimmten interessierenden Regionen des Patienten befinden, von denen vorab medizintechnische Bilddaten erfasst wurden (CT, MR, Röntgenbilder, Ultraschall, PET).

Grundsätzlich ist eine solche Navigationstechnik beispielsweise in der DE 196 39 615 C2 beschrieben worden.

Die Kombination von Durchleuchtungs- bzw. Schichtbilderfassungs-Bilddaten und Videobildern bzw. Realbildern ist grundsätzlich auch schon bekannt, beispielsweise aus der EP 1 321 105 B1 oder der EP 0 685 088 B1 und aus der Einblendung von virtuellem Bildmaterial in Bildern chirurgischer Mikroskope. Bei diesen herkömmlichen Bildausgabeverfahren wird eine Führung hauptsächlich anhand der visuell erfassbaren Daten, d.h. primär anhand einer "visuellen Realität" durchgeführt, während "virtuelle Bilddaten", nämlich Bilddaten aus vorab oder während der Navigation durchgeführten medizintechnischen Bildgebungsverfahren nur als eine zweite Informationsquelle, d.h. als sekundäre Bilddatenquelle zur Ergänzung dienen. Gemäß dem Stand der Technik wird also das visuell, also durch das menschliche Auge oder das durch Kameras oder Objektive unterstützte menschliche Auge, erfassbare Bild mit Hilfe der Bildgebungsdaten (CT, MR, Röntgen, usw.) lediglich angereichert, was einige Nachteile mit sich bringt. Zu der relativ geringfügigen Ausnutzung des virtuellen Bildmaterials kommt im Falle der Verwendung eines Mikroskops noch, dass ein Zugang zu der interessierenden Region freigelegt werden muss, was das Verfahren stärker invasiv macht. Wenn ein Endoskop verwendet wird, bildet dies ein zweites Instrument, das zusätzlich gesteuert und kontrolliert werden muss und grundsätzlich auch die Bewegungsfreiheit des Behandlungsinstruments stören kann. US-A-5749362 offenbart ein Bildausgabeverfahren und Vorrichtung mit die Merkmale der Präambeln der Ansprüche 1 und 16.

Es ist deshalb die Aufgabe der vorliegenden Erfindung, die Bildausgabe bei der medizintechnischen Navigation dahingehend zu verbessern, dass zur Verfügung stehende Bildinformationen optimal genutzt werden. Insbesondere soll auch die Ergonomie bei Navigationsverfahren verbessert und/oder die Invasivität minimiert werden.

Diese Aufgabe wird durch ein Verfahren gemäß dem Anspruch 1 sowie eine Vorrichtung gemäß dem Anspruch 16 gelöst. Die Unteransprüche definieren bevorzugte Ausführungsformen der Erfindung.

Das erfindungsgemäße Bildausgabeverfahren für die medizintechnische Navigation ist der Gattung nach eines, bei dem das Positionsverhältnis eines Instruments zu einem Patientenkörperteil ermittelt wird und auf einer Bildausgabe das Instrument und der Patientenkörperteil im richtigen Positionsverhältnis dargestellt werden, wobei die Darstellung des Patientenkörperteils einerseits auf virtuellen Bilddaten basiert, die mittels eines medizintechnischen Bildgebungsverfahrens erfasst wurden und andererseits auf Realbildern, die während der Navigation erfasst werden. Erfindungsgemäß werden auf der Bildausgabe primär und als Bildbasis die virtuellen Bilddaten angezeigt, welchen die Realbilder lediglich ergänzend und sekundär überlagert werden.

Mit anderen Worten dreht die vorliegende Erfindung die bisher bekannte Zusammenstellung der Bilddaten für die Bildausgabe gerade um. Primär wird die Navigationsunterstützung aufgrund der virtuellen Daten durchgeführt, und hierdurch nutzt die Erfindung einen technologischen Trend. Es können nämlich immer bessere (= realitätsgetreue) dreidimensionale Daten erzeugt und immer schneller und besser in Echtzeit verarbeitet werden, weil die entsprechende Daten verarbeitende Hardware immer schneller wird. Dies führt zu hochrealistischen und detailreichen virtuellen Bildausgaben. Als eine zweite und lediglich anreichernde Bildinformation wird die visuelle Realität, also Realbilder, wie sie das menschliche Auge wahrnimmt, mit den virtuellen Bilddaten korreliert und entsprechend den Notwendigkeiten oder den Wünschen des Benutzers mit ausgegeben. Mit einer solchen erfindungsgemäßen Technik lassen sich beispielsweise Instrumentenpositionen leichter bestätigen, wenn sie gegenüber einer weniger komplexen virtuellen Datensituation dargestellt werden, nämlich wenn beispielsweise die virtuellen Daten auf ein bestimmtes Niveau reduziert werden, so dass unnötige Informationen ausgeblendet werden. Außerdem können zusätzliche anatomische Einsichten (Live-Video) hinsichtlich der Instrumenten-Interaktionen bereitgestellt werden.

Weitere grundlegende Vorteile liegen in der Tatsache, dass weniger invasiv gearbeitet werden kann, weil die Basis-Navigationsinformationen, also die virtuellen Bilddaten eigentlich schon alle Informationen über das "Patienteninnere" enthalten und somit wenig direkter Sichtzugang geschaffen werden muss. Bei speziellen Ausführungsformen lässt sich die Anzahl der Instrumente reduzieren und präoperative bzw. intraoperative virtuelle Daten können gemäß den Instrumenten-Interaktionen aktualisiert werden.

Gemäß einer Ausführungsform der vorliegenden Erfindung können bei der Zusammenstellung des ausgegebenen Bildes die virtuellen Bilddaten höher gewichtet werden als die Realbilder, so dass die aus den Bildern bereitgestellte, navigationsunterstützende Information, welche auf virtuellen Bilddaten basiert, einen Anteil von mehr als 50%, insbesondere mehr als 70%, speziell mehr als 90% und bis zu 99,9% ausmacht. Die Gewichtung wird von der jeweiligen Einsatzsituation abhängen.

Die virtuellen Bilddaten können solche Bilddaten umfassen, die mittels einer Computertomographie, einer Kernspintomographie, einer Röntgen- oder Fluoroskopieaufnahme oder einer PET-, SPECT-Aufnahme oder einem anderen medizintechnischen Bildgebungsverfahren vor oder während der Navigation erfasst werden. Die Realbilder können visuell erfassbare Bilder sein, insbesondere Bilder, welche einer realen Ansicht einer interessierenden Region entsprechen oder die Realität direkt abbilden, weil sie speziell durch Videoerfassungsgeräte oder durch Objektiv umgesetzte Abbilder umfassen, die in Echtzeit erfasst werden. Mit dem Begriff "Realbilder" sind alle Bilder gemeint, wie das menschliche Auge sie sehen kann bzw. das z. B. durch Kamera oder Objektiv unterstützte menschliche Auge sie sehen kann. Sie stammen von dem betrachteten Objekt bzw. Patientenkörperteil selbst und nicht - wie die virtuellen Bilddaten - aus einem Datensatz für den Patientenkörperteil.

Wenn die erfindungsgemäße Einteilung in primäre Bildbasis und sekundäre Bildbasis eingehalten wird bzw. eine entsprechende Gewichtung erfolgt, können auch bei der vorliegenden Erfindung grundsätzlich die Realbilder durch Mikroskope oder Endoskope geliefert werden.

Erfindungsgemäß werden die Realbilder durch ein Instrument angeornete eine Videobild-Erfassungseinheit geliefert. Bei einer bevorzugten Ausführungsform der Erfindung ist dies insbesondere eine Kamera oder einen Kamera-Lichtaufnehmer (Objektiv oder Lichtleiterende). Das Instrument kann ein medizinisches Behandlungsinstrument sein, speziell ein Pointer, ein Koagulationsinstrument, eine Pinzette, ein Skalpell oder eine Klemme. Gerade bei diesen Ausführungsformen ergibt sich eine Verringerung der Instrumentenanzahl.

Das erfindungsgemäß navigierte Instrument ist vorzugsweise speziell für diese Navigation ausgestattet, insbesondere mittels eines Trackingsystems positionell erfassbar, was durch das Bereitstellen einer Trackingreferenz realisiert werden kann. Es kann auch als vorkalibriertes Instrument bereitgestellt werden, d.h. als ein Instrument, dessen Geometrie im Navigationssystem hinterlegt und vorbekannt ist und das deshalb eindeutig identifiziert und unmittelbar ohne Vorkalibrierung navigiert werden kann. Es können mehrere Instrumente verwendet werden, die jeweils mit einer Videobild-Erfassungseinheit versehen sind, wobei die so erhaltenen Realbilddaten zur gegenseitigen Beobachtung oder Überwachung der Instrumente bzw. zur ergänzenden Bilderstellung verwendet werden. Störende Interaktionen lassen sich so vermeiden. Mit der Videobild-Erfassungseinheit können auch nicht sichtbare Lichtwellenlängen erfasst werden, die dann zur Bildoptimierung verwendet werden.

Es besteht die Möglichkeit, die Kombination der virtuellen Bilddaten und der Realbilder zur positionellen Registrierung der virtuellen Bilddaten zu verwenden, insbesondere zur elastischen Bilddatenregistrierung (Morphing). Ferner kann diese Kombination zur Aktualisierung der virtuellen Bilddaten verwendet werden. Im Navigationssystem oder durch eine spezielle separate Computereinheit kann für eine Zusammenstellung des auszugebenden Bildes das Bildmaterial, d.h. die Bildinformationen, auf Relevanz geprüft (und gewichtet) werden, wobei dann unwichtigere Bildanteile aus dem Bild weggelassen und/oder wichtigere Bildanteile im Bild verstärkt oder hervorgehoben werden. Dies kann auch durch den Verwender auf dessen Eingabe hin gesteuert werden.

Die Erfindung betrifft ferner ein Programm, das, wenn es auf einem Computer läuft oder in einem Computer geladen ist, den Computer veranlasst, ein Verfahren durchzuführen, wie es hierin in verschiedenen Ausführungsformen beschrieben wird. Ferner betrifft sie noch ein Computerprogramm-Speichermedium mit einem solchen Programm.

Die Erfindung kann alle hierin beschriebenen Merkmale in jedweder sinnvollen Kombination oder auch einzeln umfassen. Sie kann insbesondere als Verfahren angesehen werden, lässt sich aber auch, wie im Anspruch 16, als Vorrichtung interpretieren. Die Verwendung der hierin aufgeführten Verfahrensmerkmale oder Vorrichtungsmerkmale im Rahmen der Bilderstellung bei der medizintechnischen Navigation ist ebenfalls Bestandteil der vorliegenden Erfindung.

Die einzige beiliegende Figur zeigt schematisch eine Ausführungsform auf, welche die vorliegende Erfindung umsetzt.

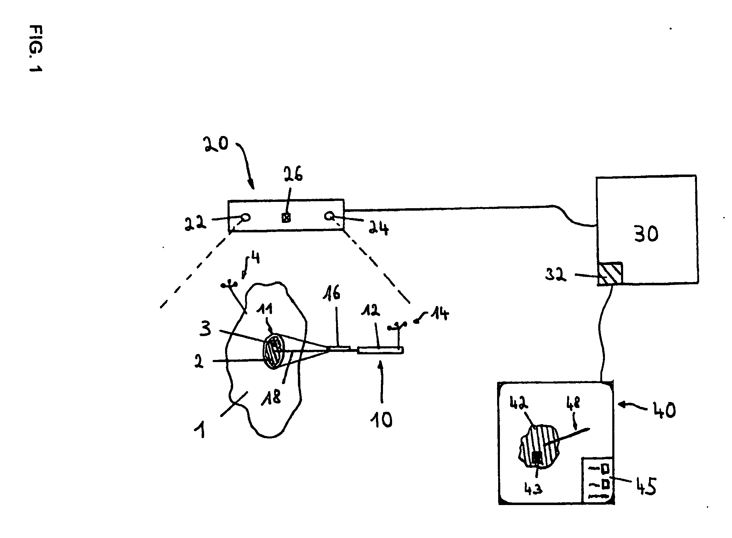

In der Figur ist mit dem Bezugszeichen 1 äußerst schematisch ein Patientenkörperteil aufgezeigt, der einen interessierenden Bereich 2 aufweist, in dem eine navigationsunterstützte Behandlung stattfinden soll. Im interessierenden Bereich 2 befindet sich Gewebe, das vorab durch eine medizinische Bildgebung untersucht worden ist und von dem ein virtueller Datensatz erstellt wurde (beispielsweise mit einem Magnetresonanz- bzw. Kernspinverfahren). Dieses Gewebe ist schraffiert dargestellt. Ferner ist im interessierenden Bereich 2 noch mit dem Bezugzeichen 3 ein Objekt abgebildet, das besonders gut visuell erkennbar ist.

Am Patientenkörperteil befindet sich eine Referenzanordnung 4. Diese Referenzanordnung 4 sowie eine Referenzanordnung am ebenfalls dargestellten chirurgischen Instrument 10 befinden sich im Sichtbereich eines Trackingsystems 20, das mit zwei Kameras 22 und 24 diese Referenzanordnungen 4 und 14 positionell erfasst und verfolgt, so dass bekannt ist, wo sich das Instrument 10 und der Körperteil 1 zu jedem Zeitpunkt befinden. Das Trackingsystem 20 sendet über den Infrarotsender 26 Infrarotblitze aus, die von Markerkugeln an den Trackingreferenzen 4, 14 reflektiert werden.

Mit dem Instrument 10, das über den Griff 12 bedient werden kann und das eine Instrumentenspitze 18 aufweist, kann ein Chirurg im interessierenden Bereich 2 arbeiten, und zwar auch minimal invasiv durch eine sehr kleine Körperöffnung hindurch, weil ihm die Bilddaten über die Positionierung der Spitze 18 seines Instruments und des Gewebes im interessierenden Bereich 2 auf einer Bildausgabe 40 bereitgestellt werden, und zwar als Gewebeabbild 42 und Spitzenabbild 48.

Die Bilderzeugung und die Steuerung der Bildausgabe erfolgen zusammen mit der notwendigen Positionsberechnung mit Hilfe eines Navigationssystems 30, das nur schematisch dargestellt wird und in dem ein Teil als "Black Box" als Bildanzeigesteuerung 32 aufgezeigt ist. Mit dieser Bildanzeigesteuerung 32 kann beispielsweise beeinflusst werden, dass als primäre und grundlegende Bilddaten virtuelle Bilddaten und erst sekundär Realbilddaten verwendet werden. Weitere Steuerungen können beispielsweise an der Bildausgabe 40 selbst vorgenommen werden, möglicherweise wie dargestellt über ein Touch-Screen-Feld 45 durch Eingaben des Verwenders des Systems. Die virtuellen Bilddaten sind im Navigationssystem 30 gespeichert, und die Positionsdaten erhält das Navigationssystem 30 vom Trackingsystem 20. Es erhält aber noch eine weitere Information, nämlich die sekundären Realbilddaten zur Erstellung des Bildes auf der Bildausgabe 40. Diese werden beim vorliegenden Ausführungsbeispiel durch eine Videokamera erzeugt, die auf der Instrumentenspitze 18 sitzt und mit dem Bezugszeichen 16 versehen worden ist. Diese Kamera hat ein Sichtfeld 11; sie kann ein Bild des gesamten interessierenden Bereiches 2 machen, insbesondere aber von dem Objekt 3.

Anders als bei bisherigen Ausführungsformen nach dem Stand der Technik wird das Abbild 42 des interessierenden Bereichs aber nicht hauptsächlich durch das Bild der Kamera 16 (welches per Funk oder Kabel zum Navigationssystem 30 übertragen werden kann) aufgebaut, denn hierfür eignet sich, wie die vorliegende Erfindung erkannt hat, besser das virtuelle Bildmaterial, d.h. die virtuellen Bilddaten aus der vorab erstellten Kernspintomographie. Das Abbild 43 des Objekts kann aber in die Bildausgabe 40 hinein "ergänzt werden" und zwar aus dem Bildmaterial, das von der Kamera 16 stammt und vielleicht gerade in dem Fall des Objekts 3 ein besseres oder aktuelleres Bild liefert. Das Abbild 43 des Objektes ist also ein sekundäres Bild in der ansonsten auf den primären, virtuellen Gewebebilddaten 42 basierenden Navigation.

Im Weiteren wird noch auf mögliche Ausführungsformen der Erfindung Bezug genommen: Beispielsweise wird ein chirurgisches Instrument, z. B. eine Pinzette zur Tumor-Ablation oder eine Blakesley-Klemme zur Entfernung von Weichgewebe, welches für die Navigation kalibriert worden ist, mit einer miniaturisierten Videokamera für die Echtzeit-Bildaufnahme ausgestattet, wobei das Sichtfeld der Kamera die Interaktionen des chirurgischen Instruments gegenüber der näheren Umgebung beobachtet. Optional - z. B. mit der Hilfe von optischen Filtern und/oder Lichtquellen, erweitert die Kamera die für den Verwender nutzbaren Lichtwellenlängen. Beispielsweise kann die Kamera fluoreszente ultraviolette Farbstoffe erfassen, welche einen kortikalen Blutstrom, Tumorgrenzen oder anderes kennzeichnen. Eine Wellenlängen-Auswahl bzw. die Verwendung spezieller Wellenlängen kann ebenfalls eingesetzt werden, um unnötige Informationen zu absorbieren oder auszusondern (z.B. Blut auf der Kameralinse).

Wenn mehr als ein Instrument verwendet wird, können auf jedem Instrument oder auf einigen Instrumenten angeordnete Kameras dazu verwendet werden, eine Videoüberwachung für die Instrumente bereitzustellen, um sicherzustellen, dass die Instrumente nicht miteinander in Konflikt geraten bzw. kollidieren. Eine weitere Einsatzmöglichkeit besteht darin, die Videobilder zusammen mit den entsprechenden 3D-Positionen für eine elastische Bildregistrierung zu verwenden, wobei die Videobilder intraoperative Bilddaten geringer Qualität (beispielsweise Ultraschall) mit zusätzlichen Informationen anreichern können, um einen besseren Ausgangspunkt für Algorithmen zu schaffen, welche Bildregistrierungen durchführen (Morphing).

Die Lokalisierung des interessierenden Bereichs kann erfindungsgemäß unter Navigationsführung durchgeführt werden, wobei entlang des Eingriffsweges die Videobilder stetig die virtuellen Bilddaten anreichern, beispielsweise transparent überlagert werden, um ein besseres anatomisches Verständnis und eine visuelle Bestätigung der antizipierten Instrumentenpositionen zu ermöglichen.

Wenn das Instrument sich in der interessierenden Region befindet, kann seine Positionierung noch immer unter Navigationsführung erfolgen, die antizipierte Navigationsposition kann noch immer visuell bestätigt werden, aber zusätzlich bietet das erfindungsgemäße System noch die Möglichkeit, die Interaktionen des Instruments mit der Kamera zu beobachten (mit anderen Instrumenten oder seiner unmittelbaren Umgebung). Außerdem können voroperative Daten angepasst werden, die sich aufgrund des Instrumenteneinsatzes ändern, wenn beispielsweise durch das Instrument Gewebe entfernt wird. Mit diesem Ansatz wird es möglich, das Volumen des entfernten Weichgewebes zu quantifizieren (beispielsweise durch Bildverarbeitungstechniken), und diese Quantifizierungen können zusammen mit den entsprechenden 3D-Positionen ebenfalls dazu verwendet werden, die schon vorher genannten elastischen Bildregistrierungen durchzuführen. Die Quantifizierungen können nämlich, auch hierbei intraoperative virtuelle Daten von geringer Qualität (Ultraschall, CT, Low-Field-MR) mit zusätzlichen Informationen anreichern, um bessere Algorithmus-Ausgangspunkte bzw. Randbedingungen für Morphing-Verfahren zu schaffen, wie sie vorher schon erläutert wurden.

## Patentansprüche

1. Bildausgabeverfahren für die medizintechnische Navigation, bei der das Positionsverhältnis eines Instruments (10) zu einem Patientenkörperteil (1) ermittelt wird, und bei dem auf einer Bildausgabe (40) das Instrument (10) und der Patientenkörperteil (1) im richtigen Positionsverhältnis dargestellt werden, wobei die Darstellung des Patientenkörperteils (1) einerseits auf virtuellen Bilddaten (42) basiert, die mittels eines medizintechnischen Bildgebungsverfahren erfasst wurden und andererseits auf Realbildern (43), die während der Navigation erfasst werden, wobei auf der Bildausgabe (40) primär und als Bildbasis die virtuellen Bilddaten (42) angezeigt werden, welchen die Realbilder (43) lediglich ergänzend und sekundär überlagert werden, **dadurch gekennzeichnet, dass** die Realbilder (43) durch eine am Instrument (10) angeordnete Videobild-Erfassungseinheit (16) geliefert werden.

2. Verfahren nach Anspruch 1, bei dem bei der Zusammenstellung des ausgegebenen Bildes die virtuellen Bilddaten höher gewichtet werden als die Realbilder.

3. Verfahren nach Anspruch 1 oder 2, bei dem die virtuellen Bilddaten solche Bilddaten umfassen, die mittels einer Computertomographie, einer Kernspintomographie, einer Röntgen- oder Fluoroskopieaufnahme, oder eine PET-, SPECT-Aufnahme oder einem anderen medizintechnischen Bildgebungsverfahren vor oder während der Navigation erfasst werden.

4. Verfahren nach einem der Ansprüche 1 bis 3, bei dem die Realbilder visuell erfassbare Bilder sind, insbesondere Bilder, welche einer realen Ansicht einer interessierenden Region entsprechen oder die Realität direkt abbilden, wobei sie speziell durch Videoerfassungsgeräte oder durch Objektiv umgesetzte Abbilder umfassen, die in Echtzeit erfasst werden.

5. Verfahren nach einem der Ansprüche 1 bis 4, bei dem die Realbilder durch ein Mikroskop oder ein Endoskop geliefert werden.

6. Verfahren nach einem der Ansprüche 1 bis 4, bei dem die Realbilder durch eine eine Kamera oder einen Kamera-Lichtaufnehmer, geliefert werden, die an dem Instrument (10) angeordnet ist, welches insbesondere ein medizinisches Behandlungsinstrument ist, speziell ein Pointer, eine Pinzette, ein Skalpell oder eine Klemme.

7. Verfahren nach einem der Ansprüche 1 bis 6, bei dem das Instrument (10) ein für die medizintechnische Navigation ausgestattetes Instrument (10) ist, insbesondere ein mittels eines Trackingsystems (20) positionell erfassbares, speziell mit einer Trackingreferenz (14) versehenes Instrument.

8. Verfahren nach einem der Ansprüche 1 bis 7, bei dem das Instrument (10) ein zur Navigation mit einem verwendeten Navigationssystem (30) vorkalibriertes Instrument ist.

9. Verfahren nach einem der Ansprüche 6 bis 8, bei dem mehrere Instrumente verwendet werden, die jeweils mit einer Videobild-Erfassungseinheit (16) versehen sind, wobei die so erhaltenen Realbilddaten zur gegenseitigen Beobachtung oder Überwachung der Instrumente bzw. zur ergänzenden Bilderstellung verwendet werden.

10. Verfahren nach einem der Ansprüche 6 bis 9, bei dem mit der Videobild-Erfassungseinheit (16) auch nicht sichtbare Lichtwellenlängen erfasst werden, die dann zur Bildoptimierung verwendet werden.

11. Verfahren nach einem der Ansprüche 1 bis 10, bei dem die Kombination der virtuellen Bilddaten und der Realbilder zur positionellen Registrierung der virtuellen Bilddaten verwendet wird, insbesondere zur elastischen Bilddatenregistrierung.

12. Verfahren nach einem der Ansprüche 1 bis 11, bei dem die Kombination der virtuellen Bilddaten und der Realbilder zur Aktualisierung der virtuellen Bilddaten verwendet wird.

13. Verfahren nach einem der Ansprüche 1 bis 12, bei dem bei der Zusammenstellung des auszugebenden Bildes Bildinformationen auf Relevanz geprüft und gewichtet werden, wobei dann unwichtigere Bildanteile aus dem Bild weggelassen und/oder wichtigere Bildanteile im Bild verstärkt oder hervorgehoben werden.

14. Programm, das, wenn es auf einem Computer läuft oder in einem Computer geladen ist, den Computer veranlasst, ein Verfahren gemäß einem der Ansprüche 1 bis 13 durchzuführen.

15. Computerprogramm-Speichermedium, das ein Programm nach Anspruch 14 aufweist.

16. Bilderzeugungs- und Bildausgabevorrichtung für die medizintechnische Navigation, bei der das Positionsverhältnis eines Instruments (10) zu einem Patientenkörperteil (1) ermittelt wird, mit einer Bildausgabe (40), auf der das Instrument (10) und der Patientenkörperteil (1) im richtigen Positionsverhältnis dargestellt werden, einer Bildverarbeitung, welche eine Darstellung des Patientenkörperteils (1) einerseits auf der Basis von virtuellen Bilddaten (42) erzeugt, die mittels eines medizintechnischen Bildgebungsverfahren erfasst wurden, und andererseits auf der Basis von Realbildern (43), die während der Navigation erfasst werden, und einer Bildanzeigesteuerung (32), die auf der Bildausgabe (40) primär und als Bildbasis die virtuellen Bilddaten (42) anzeigt, welchen die Realbilder (43) lediglich ergänzend und sekundär überlagert werden, **gekennzeichnet durch**
- eine an dem Instrument (10) angeordnete Videobild-Erfassungseinheit (16), welche die Realbilder liefert.

## Claims

1. An image output method for medical navigation in which the positional relationship of an instrument (10) with respect to a part (1) of a patient's body is ascertained, and in which the instrument (10) and the part (1) of the patient's body are displayed in the correct positional relationship on an image output (40), wherein the display of the part (1) of the patient's body is based on the one hand on virtual image data (42) captured by means of a medical imaging method and on the other hand on actual images (43) captured during navigation, wherein the virtual image data (42) are displayed on the image output (40) primarily and as the basis of the image, and the actual images (43) are superimposed on them merely as an addition and secondarily, **characterised in that** the actual images (43) are provided by a video image capture unit (16) which is arranged on the instrument (10).

2. The method according to claim 1, wherein the virtual image data are weighted more heavily than the actual images when assembling the outputted image.

3. The method according to claim 1 or 2, wherein the virtual image data comprise image data which are captured before or during navigation by means of computed tomography, magnetic resonance tomography, an x-ray recording or fluoroscopic recording, a PET or SPECT recording or another medical imaging method.

4. The method according to any one of claims 1 to 3, wherein the actual images are images which can be visually captured, in particular images which correspond to an actual view of a region of interest or which directly image the reality, wherein they comprise images which are implemented specifically by video capture apparatus or by an object lens and captured in real time.

5. The method according to any one of claims 1 to 4, wherein the actual images are provided by a microscope or an endoscope.

6. The method according to any one of claims 1 to 4, wherein the actual images are provided by a camera or camera light recorder which is arranged on the instrument (10) which is in particular a medical treatment instrument, specifically a pointer, a pair of tweezers, a scalpel or a clamp.

7. The method according to any one of claims 1 to 6, wherein the instrument (10) is an instrument (10) which is equipped for medical navigation, in particular an instrument which can be positionally detected by means of a tracking system (20) and which is specifically provided with a tracking reference (14).

8. The method according to any one of claims 1 to 7, wherein the instrument (10) is an instrument which is pre-calibrated for navigation with a navigation system (30) used.

9. The method according to any one of claims 6 to 8, wherein a plurality of instruments are used which are each provided with a video image capture unit (16), wherein the actual image data thus obtained are used to reciprocally observe or monitor the instruments and/or to produce supplementary images.

10. The method according to any one of claims 6 to 9, wherein non-visible light wavelengths are also detected using the video image capture unit (16) and are then used for image optimisation.

11. The method according to any one of claims 1 to 10, wherein the combination of the virtual image data and the actual images is used to positionally register the virtual image data, in particular for elastic image data registration.

12. The method according to any one of claims 1 to 11, wherein the combination of the virtual image data and the actual images is used for updating the virtual image data.

13. The method according to any one of claims 1 to 12, wherein image information for assembling the image to be outputted is tested for relevance and weighted, wherein less important image constituents are then omitted from the image and/or more important image constituents are intensified or highlighted in the image.

14. A program which, when it is running on a computer or is loaded on a computer, causes the computer to perform a method in accordance with any one of claims 1 to 13.

15. A computer program storage medium comprising a program according to claim 14.

16. An image generating and image output device for medical navigation in which the positional relationship of an instrument (10) with respect to a part (1) of a patient's body is ascertained, comprising: an image output (40) on which the instrument (10) and the part (1) of the patient's body are displayed in the correct positional relationship; an image processor which generates a display of the part (1) of the patient's body on the basis of virtual image data (42) captured by means of a medical imaging method on the one hand and on the basis of actual images (43) captured during navigation on the other hand; and an image display control (32) which displays the virtual image data (42) on the image output (40) primarily and as the basis of the image, wherein the actual images (43) are superimposed on the virtual image data merely as an addition and secondarily, **characterised by**:
- a video image capture unit (16) which is arranged on the instrument (10) and provides the actual images.

## Revendications

1. Procédé de sortie d'image pour la navigation médicale, dans lequel la relation de position d'un instrument (10) par rapport à une partie corporelle d'un patient (1) est déterminée, et dans lequel l'instrument (10) et la partie corporelle de patient (1) sont affichés dans la relation de position correcte sur une sortie d'image (40), dans lequel l'affichage de la partie corporelle de patient (1) est basé d'une part sur des données d'images virtuelles (42) qui ont été capturées au moyen d'un procédé d'imagerie médicale, et d'autre part sur des images réelles (43) qui ont été capturées au cours de la navigation, dans lequel les données d'images virtuelles (42) sont affichées principalement sur la sortie d'image (40) et en tant que base d'image, données d'images virtuelles auxquelles sont superposées les images réelles (43) uniquement en complément et de manière secondaire, **caractérisé en ce que** les images réelles (43) sont fournies par une unité de capture d'image vidéo (16) agencée sur l'instrument (10).

2. Procédé selon la revendication 1, dans lequel lors de l'assemblage de l'image de sortie, les données d'images virtuelles sont pondérées plus fortement que les images réelles.

3. Procédé selon la revendication 1 ou 2, dans lequel les données d'images virtuelles incluent des données d'images qui sont capturées au moyen d'une tomographie assistée par ordinateur, d'une tomographie par résonance magnétique nucléaire, d'un enregistrement aux rayons X ou d'un enregistrement par fluoroscopie, ou d'un enregistrement PET ou SPECT, ou d'un autre procédé d'imagerie médicale avant ou pendant la navigation.

4. Procédé selon l'une des revendications 1 à 3, dans lequel les images réelles sont des images pouvant être capturées visuellement, en particulier des images qui correspondent à une vue réelle d'une région présentant un intérêt ou qui reproduisent directement la réalité, dans lequel elles incluent des images spécialement mises en oeuvre par des appareils de capture vidéo ou par une lentille d'objectif, lesquelles images sont capturées en temps réel.

5. Procédé selon l'une des revendications 1 à 4, dans lequel les images réelles sont fournies par un microscope ou un endoscope.

6. Procédé selon l'une des revendications 1 à 4, dans lequel les images réelles sont fournies par une caméra ou un enregistreur de luminosité de caméra qui est agencé sur l'instrument (10), lequel instrument est notamment un instrument de traitement médical, en particulier un pointeur, des pinces, un scalpel ou un clamp.

7. Procédé selon l'une des revendications 1 à 6, dans lequel l'instrument (10) est un instrument (10) équipé pour la navigation médicale, en particulier un instrument spécialement muni d'une référence de repérage (14) dont la position peut être détectée au moyen d'un système de repérage (20).

8. Procédé selon l'une des revendications 1 à 7, dans lequel l'instrument (10) est un instrument pré-calibré pour la navigation avec un système de navigation utilisé (30).

9. Procédé selon l'une des revendications 6 à 8, dans lequel sont utilisés plusieurs instruments qui sont chacun munis d'une unité de capture d'image vidéo (16), dans lequel les données d'images réelles ainsi obtenues sont utilisées pour observer ou surveiller réciproquement les instruments ou pour produire des images complémentaires.

10. Procédé selon l'une des revendications 6 à 9, dans lequel des longueurs d'onde non visibles sont également détectées avec l'unité de capture d'image vidéo (16), lesquelles longueurs d'onde sont ensuite utilisées pour l'optimisation d'image.

11. Procédé selon l'une des revendications 1 à 10, dans lequel la combinaison des données d'images virtuelles et des images réelles est utilisée pour enregistrer des positions des données d'images virtuelles, en particulier pour enregistrer des données d'images élastiques.

12. Procédé selon l'une des revendications 1 à 11, dans lequel la combinaison des données d'images virtuelles et des images réelles est utilisée pour actualiser les données d'images virtuelles.

13. Procédé selon l'une des revendications 1 à 12, dans lequel lors de l'assemblage de l'image à sortir, la pertinence des informations d'image est testée et pondérée, dans lequel des portions d'image moins importantes sont ensuite omises de l'image et/ou des portions d'image plus importantes sont intensifiées ou accentuées dans l'image.

14. Programme qui, lorsqu'il s'exécute sur un ordinateur ou lorsqu'il est chargé dans un ordinateur, amène l'ordinateur à mettre en oeuvre un procédé selon l'une quelconque des revendications 1 à 13.

15. Support de mémorisation de programme informatique comportant un programme selon la revendication 14.

16. Dispositif de génération d'image et de sortie d'image pour la navigation médicale, dans lequel la relation de position d'un instrument (10) par rapport à une partie corporelle de patient (1) est déterminée, comportant une sortie d'image (40) sur laquelle l'instrument (10) et la partie corporelle de patient (1) sont affichés dans une relation de position correcte, une unité de traitement d'image qui génère un affichage de la partie corporelle de patient (1) d'une part sur la base de données d'images virtuelles (42) qui ont été capturées au moyen d'un procédé d'imagerie médicale, et d'autre part sur la base d'images réelles (43) qui ont été capturées pendant la navigation, et une commande d'affichage d'image (32) qui affiche principalement les données d'images virtuelles (42) sur la sortie d'image (40) et en tant que base d'image, données d'images virtuelles auxquelles sont superposées les images réelles (43) uniquement en complément et de manière secondaire,
**caractérisé par**
- une unité de capture d'image vidéo (16) disposée sur l'instrument (10), qui fournit les images réelles.
